# EUROPEAN PATENT APPLICATION

(11) **EP 4 528 569 A1**
(43) Date of publication of application: **26.03.2025**
(21) Application number: 24197891.5
(22) Date of filing: 02.09.2024
(51) Int. Cl.: G06F 21/64, G16H 10/60, H04L 9/08, H04L 9/32, H04L 9/00, H04L 9/40, G16H 15/00, G16H 80/00

(54) **SYSTEM AND METHOD FOR IMPLEMENTING MEDICAL DATA EXCHANGE AND MEDICAL CLAIM SETTLEMENT**

(30) Priority: 19.09.2023 TW 112135699
(71) Applicant: Cathay Financial Holding Co., Ltd., Taipei City 106 (TW)
(72) Inventor: CHIANG, Kun-Cheng, 106 Taipei City (TW); KUNG, Yun-Ting, 106 Taipei City (TW); JHANG, Fang-Yu, 106 Taipei City (TW); LIAO, Ming-Hung, 106 Taipei City (TW)
(74) Representative: Lang, Christian

(57) **Abstract**

Disclosed herein is a system for implementing medical data exchange and medical claim settlement via Fast Healthcare Interoperability Resources (FHIR) standard data. The system includes a data exchange module, a blockchain database, a service-provider server, and an authority management module, all of which are communicatively connected to each other. The system receives and converts raw medical data into FHIR standard data for transmission to insurance institutions, and encrypts the FHIR standard data, and facilitates medical claims settlement. Also disclosed herein is a method for implementing medical data exchange and claims settlement based on FHIR standards.

## Description

### CROSS-REFERENCES TO RELATED APPLICATIONS

This application claims priority and the benefit of Taiwan Patent Application No. 112135699, filed September 19, 2023, the entireties of which is incorporated herein by reference.

### BACKGROUND OF THE INVENTION

### 1. FIELD OF THE INVENTION

The present disclosure relates to a system and method for exchanging data and providing commercial services. More particularly, the disclosed invention relates to a system and method for achieving medical data exchange and medical claim settlement based on Fast Healthcare Interoperability Resources (FHIR) standard.

### 2. DESCRIPTION OF RELATED ART

Fast Healthcare Interoperability Resources (FHIR) is a standard concerning patient data rights recently issued by the U.S. government mandating healthcare institutions exchange medical data concerning information such as patient records, admission and discharge notes, and referral documents, must adhere to a unified standard. The purpose of FHIR is to enhance the interoperability of medical information between institutions.

The differences in field names, definitions, and corresponding value formats across different healthcare institutions create difficulties in data integration for insurance companies upon reviewing medical claims. As a result, claims handler must manually interpret and adjust the formats one by one before the data can be successfully integrated into the system for further claims processing. This not only increases the burden on claims handler but also prolongs the approval process, causing unnecessary waiting time of policyholders from several weeks to months before receiving their claim payments. This delay also affects policyholders' trust in the insurance companies.

Additionally, due to the sensitive nature of medical data, personal and medical information cannot be converted using public applications or online tools, making data integration even more challenging.

In view of the foregoing, there exists in the related art a need for an improved system for medical information exchange and claims processing.

### SUMMARY

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

The first aspect of the present disclosure is directed to a system communicatively connected to a server for implementing medical data exchange and medical claim settlement via a Fast Healthcare Interoperability Resources (FHIR) standard data, wherein the server comprises a plurality of raw medical data of a first format stored therein. According to embodiments of the present disclosure, the present system comprises a data exchange module, a blockchain database, a service-provider server, and an authority management module. The data exchange module is configured to receive the plurality of raw medical data from the server and convert them into a plurality of FHIR authorized data in response to a mapping instruction, using a built-in converter in the data exchange module; the blockchain database is communicatively connected to the data exchange module and is configured to store the plurality of FHIR authorized data; the service-provider server is communicatively connected to both the data exchange module and the blockchain database, and is configured to provide claim settlement based on the plurality of FHIR authorized data; and the authority management module is communicatively connected to the data exchange module and the service-provider server, and is programmed with instructions to execute a method for managing each of the FHIR authorized data transmitted among the data exchange module, the blockchain database, and the service-provider server. In the present system, the method comprises the steps of: (a) receiving a first public key and a first private key from the server, and a second public key and a second private key from the service-provider server, respectively; (b) combining the first private key and the second public key from step (a), thereby producing a key; (c) encrypting each of the FHIR authorized data with the key produced in step (b), thereby producing a ciphered FHIR authorized data; (d) providing a signature to the ciphered FHIR authorized data from step (c) by use of the first private key, thereby producing a signed FHIR authorized data; (e) verifying the signed FHIR authorized data from step (d) by use of the first public key to validate and rescind the signature, thereby restoring the ciphered FHIR authorized data from step (c); and (f) decrypting the ciphered FHIR authorized data of step (e) with the key from step (b) to obtain the FHIR authorized data that has been decrypted, wherein the key is produced by combining the second private key and the first public key of step (a).

According to embodiments of the present disclosure, the FHIR standard data has a second format that differs from the first format of the plurality of raw medical data. According to one embodiment of the present disclosure, the mapping instruction is produced through self-defining the FHIR standard data of the second format. According to one embodiment of the present disclosure, the first format is an extensible markup language (XML) format, and the second format is a JavaScript object notation (JSON) format. In preferred embodiments of the present disclosure, the converter is an XML to JSON converter.

According to embodiments of the present disclosure, in the present system, the method executed by the authority management module further comprises transmitting the ciphered FHIR authorized data from step (c), the signed FHIR authorized data from step (d), or a combination thereof to the blockchain database after the ciphered FHIR authorized data and/or the signed FHIR authorized data are produced.

Alternatively, or optionally, in the present system, the method set forth above further comprises steps of (i) calculating the ciphered FHIR authorized data to produce a hash value, and (ii) transmitting the hash value to the blockchain database.

In alternative embodiments of the present disclosure, the service-provider server of the present system further comprises a data verification unit that is communicatively connected to the blockchain database and is configured to calculate the ciphered FHIR authorized data to produce a checksum, and to verify the consistency between the hash value and the checksum.

The second aspect of the present disclosure is directed to a computer-implemented method for exchanging medical data and settling medical claims for a client using the client's raw medical data via the present system set forth above substantially based on FHIR standard. According to embodiments of the present disclosure, the present method comprises: (i) receiving a raw medical data of the client from the server, wherein the raw medical data has a first format; (ii) converting the raw medical data to a FHIR authorized data in response to a mapping instruction by the converter of the present system, in which the FHIR standard data has a second format that differs from the first format of the raw medical data, and the FHIR authorized data is stored in the blockchain database of the present system; and (iii) providing a medical claim service to the client based on the FHIR authorized data of step (ii) by the service-provider server of the present system. More specifically, when the FHIR authorized data is transmitted among the data exchange module, the blockchain database, and the service-provider server of the present system, the FHIR authorized data is processed by instructions programmed within the authority management module of the present system by executing a procedure comprising the following steps: (a) receiving a first public key and a first private key from the server, and a second public key and a second private key from the service-provider server of the present system, respectively; (b) combining the first private key and the second public key from step (a), thereby producing a key; (c) encrypting the FHIR authorized data with the key produced in step (b), thereby producing a ciphered FHIR authorized data; (d) providing a signature to the ciphered FHIR authorized data of step (c) by use of the first private key, thereby producing a signed FHIR authorized data; (e) verifying the signed FHIR authorized data of step (d) by use of the first public key to validate and rescind the signature, thereby restoring the ciphered FHIR authorized data of step (c); and (f) decrypting the ciphered FHIR authorized data of step (e) with the key of step (b) to obtain the FHIR authorized data that has been decrypted, wherein the key is produced by combining the second private key and the first public key of step (a).

According to embodiments of the present disclosure, the mapping instruction of step (ii) is produced through self-defining the FHIR standard data with the second format.

According to alternative embodiments of the present disclosure, the authority management module of the present system is further programmed with instructions to execute the steps of: transmitting the ciphered FHIR authorized data of step (c), the signed FHIR authorized data of step (d), or a combination thereof to the blockchain database after the ciphered FHIR authorized data and/or the signed FHIR authorized data are produced.

Additionally, in some embodiments of the present disclosure, the authority management module is further programmed with instructions to execute the steps of calculating the ciphered FHIR authorized data to produce a hash value and transmitting the hash value to the blockchain database after the step (c).

In some alternative embodiments of the present disclosure, the service-provider server of the present system further comprises a data verification unit that is communicatively connected to the blockchain database and is configured to calculate the ciphered FHIR authorized data to produce a checksum, and to verify the consistency between the hash value and the checksum.

Many of the attendant features and advantages of the present disclosure will becomes better understood with reference to the following detailed description considered in connection with the accompanying drawings.

### BRIEF DESCRIPTION OF THE DRAWINGS

The present description will be better understood from the following detailed description read in light of the accompanying drawings, where:
FIG. 1 is a schematic diagram of a system 100 according to one embodiment of the present disclosure;
FIG. 2 is a flow chart depicting managing steps of a method 200 performed by the authority management module 140 of the system 100 in accordance with one embodiment of the present disclosure;
FIG. 3 is a schematic diagram of a system 300 according to one alternative embodiment of the present disclosure; and
FIG. 4 is a flow chart of a method 400 for settling medical claims to a client according to one embodiment of the present disclosure.

In accordance with common practice, the various described features/elements are not drawn to scale but instead are drawn to best illustrate specific features/elements relevant to the present invention. Also, like reference numerals and designations in the various drawings are used to indicate like elements/parts.

### DESCRIPTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

### 1. Definition

For convenience, certain terms employed in the specification, examples and appended claims are collected here. Unless otherwise defined herein, scientific and technical terminologies employed in the present disclosure shall have the meanings that are commonly understood and used by one of ordinary skill in the art. Also, unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Also, as used herein and in the claims, the terms "at least one" and "one or more" have the same meaning and include one, two, three, or more.

The terms "service-provider" or "client" as used herein refers to any computer device capable of communicatively connecting to at least one server via wired or wireless networks. According to one embodiment of the present disclosure, the "service-provider" or "client" encompasses at least one graphical display device and graphical user interfaces (GUIs) that allow users to view information and interact through applications, tools, services, or software on the GUIs. In the present disclosure, the user corresponding to the "service-provider" is an insurance claim handler or a manager, while the user corresponding to the "client" is a policyholder.

The term "server" as used herein refers to a storage unit and communication unit possessing at least some form of storage capability. The storage unit includes electrically dependent and non-electrically dependent, removable and non-removable media. Appropriate methods or technologies can be used to enable the aforementioned media to store the desired information (such as computer-readable instructions, data structures, application modules, and other data). Storage media include, but are not limited to, random access memory (RAM), read-only memory (ROM), electronically erasable programmable read-only memory (EEPROM), flash memory, compact disc read-only memory (CD-ROM), digital versatile disc (DVD), or other optical storage; magnetic cassettes, tapes, disk storage, and other magnetic storage devices; or any other media capable of storing the required information and accessible to the processor. Generally, the communication unit can encode computer-readable instructions, data, structures, application modules, and other data into various data signals and transmit them through any communication media. Exemplary communication media include, but are not limited to, wired media (such as a wired network or direct wired connection) and wireless media (such as audio, infrared, radio, microwave, spread spectrum technology, and other wireless technologies).

As used herein, the term "Fast Healthcare Interoperability Resources (FHIR)" refers to the electronic medical information exchange standards published by Health Level Seven International, aimed at improving data communication and operability between different medical systems. Specifically, FHIR standardizes the structure of clinical and non-clinical data (such as electronic medical records, clinical records, imaging data, prescriptions, and patient information) by defining the fields, attributes, and relationships within the data. This standardization ensures that each field has a specific data type and format, allowing different systems to exchange and share medical information according to a consistent structure and standards, thereby facilitating data exchange between medical institutions and insurance organizations.

The term "authority management" as used herein refers to a program that organizes and manages access to data, systems, or resources, with the purpose of ensuring that only authorized users or entities can access specific resources, functions, or data. Generally, the "authority management" includes user identification and verification, granting authority, authority review and monitoring, and hierarchical management of authority levels. In one exemplary embodiment of the present disclosure, the present system manages access rights to medical information through encrypted and signed transmissions.

The term "hash" or "hash value" as used herein, refers to a mathematical function used to verify data integrity by converting input data into a fixed-length string (*i.e.,* a hash value). Generally, when using the same hash function, only identical content produces the same hash value; different content cannot generate the same hash value. Therefore, by comparing the two hash values, the consistency of the data can be verified.

### 2. Description of the invention

The current issue is that the different formats of medical data prevent insurance systems from automatically importing the information, necessitating additional manpower to adjust the format. To address this issue, this invention aims to provide a novel medical data exchange system and method that facilitate the exchange of medical data between different institutions based on the unified specifications set by FHIR, while maintaining the privacy of medical data.

### 2.1 Medical data exchange system and encryption procedures for the exchange

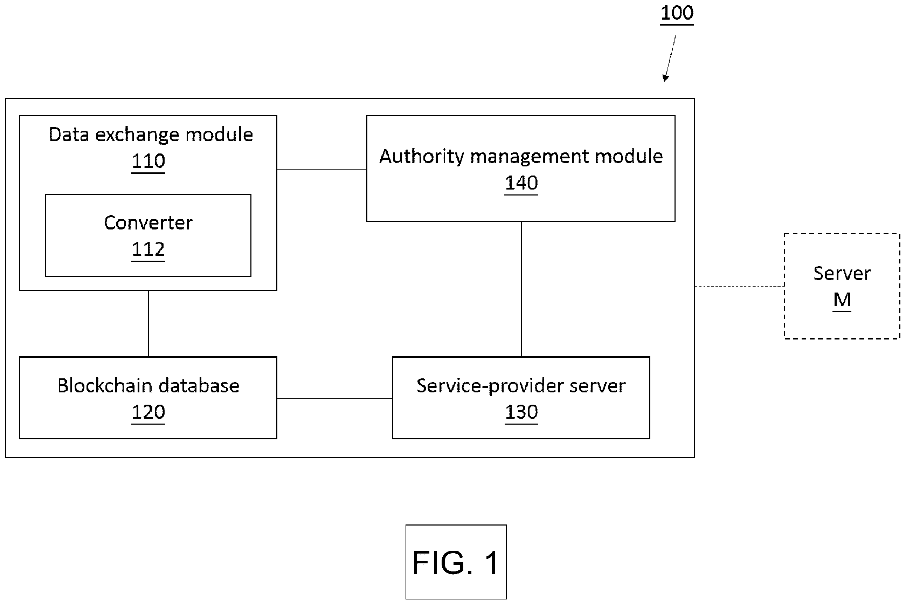

Accordingly, one aspect of the present invention is directed to a system for implementing medical data exchange and medical claim settlement via a Fast Healthcare Interoperability Resources (FHIR) standard data. Referring to FIG. 1, a system 100, which is communicatively connected to a server M, is depicted according to one embodiment of the present disclosure. As depicted, the present system 100 comprises in its structure a data exchange module 110, a blockchain database 120, a service-provider server 130, and an authority management module 140. Specifically, the data exchange module 110, the blockchain database 120 and the service-provider server 130 are communicatively connected to each other, the server M is communicatively connected to the data exchange module 110, and the authority management module 140 is communicatively connected to the data exchange module 110 and the blockchain database 120, respectively. The configuration aforementioned enables the transmission of the raw medical data having a first format from the server M to the present system 100, where the raw data is converted to comply with FHIR standard. The converted data is then provided to the service-provider server 130 (e.g., insurance company) and serves as the basis for providing medical claims services to the client.

According to embodiments of the present disclosure, the server M stores a plurality of raw medical data, which refers to the electronic medical records created by each institution or unit to record a patient's personal information (e.g., name, gender, age, marriage, type, ethnicity, occupation, place of birth, current address, workplace, phone number, etc.), past medical history, family medical history, current medical history (e.g., current symptoms, pain, discomfort, etc.), physical examination items, laboratory examination items, diagnostic results, treatment plans, medical records (e.g., date of treatment, start time of treatment, physician's name, description of diagnosis and treatment process, examination orders, prescriptions, etc.), medical opinions, follow-up records, hospitalization records (if any ), and other contents, but are not limited to these. Different medical institutions and/or hospitals may adjust the configuration of medical record items according to their practical needs.

Additionally, the raw medical data, depending on the diagnosis and examination from different specialties, can be presented as text, image data, audio recordings, or video recordings. This data is individually, separately stored in a format (i.e., first format) that can be used for sharing across different applications within the server M at medical institutions and/or hospitals, allowing physicians to review and add new medical records. According to optional embodiments of the present disclosure, when the raw medical data is in text form, the first format may be any format selected from the group consisting of Extensible Markup Language (XML), JavaScript object notation (JSON), YAML Ain't Markup Language (YAML), Resource Description Framework (RDF), Terse RDF Triple Language (Turtle), Comma-Separated Values (CSV), HyperText Markup Language (HTML), and Rich Text Format (RTF) . In the case when the raw medical data is in image form, then the first format may be Joint Photographic Experts Group (JPEG), Portable Network Graphics (PNG), Graphics Interchange Format (GIF), Bitmap (BMP), Scalable Vector Graphics (SVG), or Tagged Image File Format (TIFF). If the raw medical data is in audio form, examples of the first format may include, but is not limited to, Moving Picture Experts Group Audio Layer III (MP3), Waveform Audio File Format (WAV), Free Lossless Audio Codec (FLAC), Advanced Audio Coding (AAC), Audio Interchange File Format (AIFF), Adaptive Multi-Rate Audio Codec (AMR), and the like. In the case when the raw medical data is in video form, the first format may be Audio Video Interleave (AVI), Matroska Video (MKV), QuickTime Movie (MOV), Windows Media Video (WMV), Flash Video (FLV), Moving Picture Experts Group (MPEG), or Video Object (VOB). In one embodiment of the present disclosure, the raw medical data is in text form, and the first format may be any format selected from a group consisting of XML, JSON, RDF, and Turtle. In working examples of the present disclosure, XML is used as the first format to record the structure and content of the raw medical data.

The data exchange module 110 is communicatively connected to the server M and is configured to receive and convert the raw medical data in the first format stored in server M into FHIR authorized data. Noted that the data exchange module 110 includes a built-in converter 112 configured to carry out the conversion process. The converter 112 is a tool or program used to convert one data format into another. The conversion process is not limited to converting only the data storage format; it can also map specific items from the raw medical data to corresponding fields defined in the FHIR standard, with or without changing the storage format, based on at least one mapping instructions. In one working example, the converter 112 is an XML-to-JSON converter.

According to some embodiments of the present disclosure, when the converter 112 receives the raw medical data, it reads the data content and parses the XML format into an internal representation. Then, the converter 112 maps each element or item of the raw medical data to the corresponding fields defined in the FHIR standard in accordance with predetermined mapping rules. The format conversion is then carried out, including steps such as rearranging data, transforming it, and renaming labels, to generate FHIR authorized data complying with the target format, while incorporating the content converted from the raw medical data. The converted data can be saved to a database or any designated storage unit, and/or directly output to other medical or insurance institutions' systems or applications for use. According to optional embodiments of the present disclosure, the FHIR authorized data is directly output to the service-provider server 130 for use, while simultaneously being stored in the blockchain database 120.

The mapping instructions are used to determine the correspondence between two different data sets to facilitate data conversion, integration, or matching. In the present disclosure, the mapping instructions are self-defined based on FHIR authorized data in the second format.

Specifically, the FHIR standard data format (e.g., JSON) is parsed to decompose it into resources and elements, facilitating the management of the composition of elements within each resource and defining their field attributes and corresponding values. This process helps determine the correspondence between items in the raw medical data and the fields defined in the FHIR standards, thereby generating the mapping instructions. In the present disclosure, a resource refers to the basic unit of medical data, including any medical-related information, such as patient information, medical events, clinical observations; and elements are the related pieces of information within the resource hierarchy. For example, under the "Patient Information" resource hierarchy, examples of elements include, but are not limited to, name, gender, age, occupation, place of birth, and so on.

According to certain embodiments of the present disclosure, the second format is similar to the first format, with both being selected based on the file type for compatibility with various applications or systems for data exchange. Take text information as an example, the second format may be any format selected from the group consisting of XML, JSON, RDF, and Turtle. In non-limiting embodiments, the first and second formats can each be selected from the same group consisting of XML, JSON, RDF, and Turtle, meaning the first and second formats can be either the same or different. According to one preferred embodiment of the present disclosure, the first format is XML and the second format is JSON.

As blockchain technology is advantageous in terms of data security, transparency, decentralization, and tamper resistance, the present disclosure constructs a blockchain database 120 for storing FHIR authorized data. As depicted in FIG. 1, the blockchain database 120 is communicatively connected to the data exchange module 110 and is configured to receive and store the FHIR authorized data with a second format for subsequent queries and operations.

In addition to be transmitted to the blockchain database 120 for storage, the FHIR authorized data can also be directly transmitted to the service-provider server 130, which is communicatively connected to the data exchange module 110. This allows the service provider, either through its systems (such as the claims settlement system of an insurance organization) or personnel (e.g., claims handlers), to generate and provide claims services based on the FHIR-authorized data directly received from the data exchange module 110 or by accessing the FHIR-authorized data stored in the blockchain database 120.

According to embodiments of the present disclosure, the present system 100 provided herein is for medical data exchange. Therefore, the claims services mentioned above refer to medical-related claims, including those for treatment, hospitalization, surgery, medication, outpatient care, rehabilitation, disability, and/or critical illness.

To ensure the confidentiality and security of medical information, which contains personal and sensitive data, and to prevent potential insurance fraud, the present system 100 implements an authority management module 140 that communicatively connected to the data exchange module 110 and the service-provider server 130 to manage access rights to FHIR authorized data. According to embodiments of the present disclosure, the authority management module 140 is programmed with instructions to execute a method for managing the FHIR authorized data when it is transmitted among the data exchange module 110, the blockchain database 120, and the service-provider server 130.

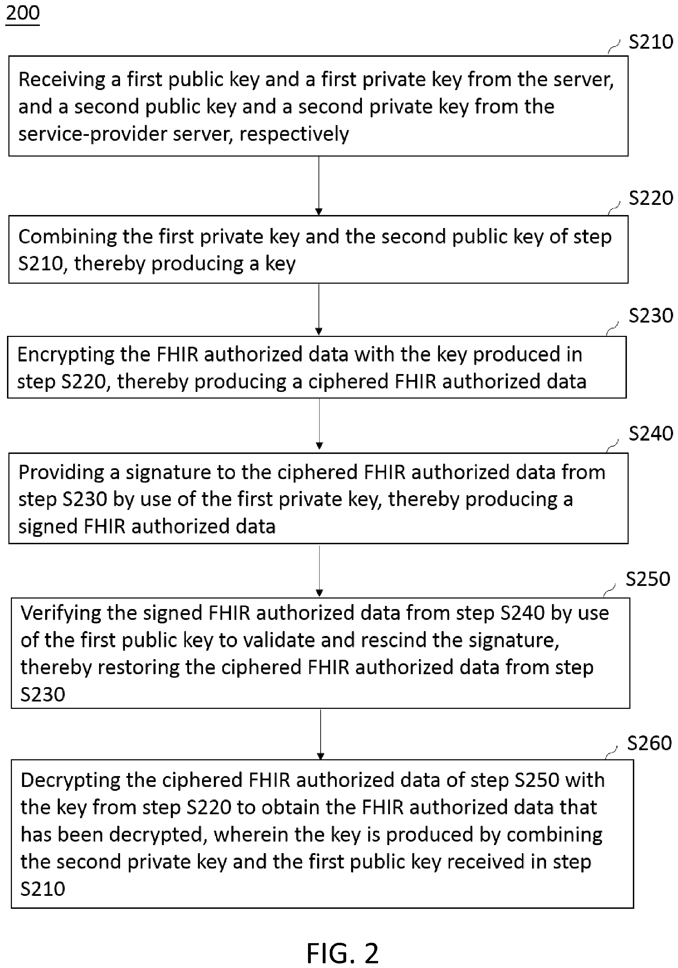

Reference is made to both FIGs. 1 and 2 for the operation of the authority management module 140. FIG.2 depicts a flow chart of the method 200 for managing the FHIR authorized data, in which the authority management module 140 is configured to execute following steps (see the reference numbers S210 to S260 indicated in FIG. 2):
S210: receiving a first public key and a first private key from the server M, and a second public key and a second private key from the service-provider server 130, respectively;
S220: combining the first private key and the second public key of step S210, thereby producing a key;
S230: encrypting the FHIR authorized data with the key produced in step S220, thereby producing a ciphered FHIR authorized data;
S240: providing a signature to the ciphered FHIR authorized data from step S230 by use of the first private key, thereby producing a signed FHIR authorized data;
S250: verifying the signed FHIR authorized data from step S240 by use of the first public key to validate and rescind the signature, thereby restoring the ciphered FHIR authorized data from step S230; and
S260: decrypting the ciphered FHIR authorized data of step S250 with the key from step S220 to obtain the FHIR authorized data that has been decrypted, wherein the key is produced by combining the second private key and the first public key received in step S210.

The present method 200 begins by receiving two sets of public and private keys: one set from server M and the other from the service-provider server 130 (S210). Generally, medical or insurance institutions using the present system 100 independently have their own set of public and private keys for encrypting files intended to be exchanged. The public key is circulated within the data exchange platform or system, while the private key is securely maintained by each institution. The authority management module 140 of the present system 100 performs encryption, decryption, and signature verification by combining the public and/or private keys of the sender and receiver. This process ensures the correctness of the data source and manages access rights to the FHIR authorized data. Accordingly, in step S210, the first public and private keys representing server M (e.g., a hospital) is provided, and the second public and private keys representing the service-provider server 130 (e.g., an insurance company) for subsequent encryption-decryption steps.

In step S220, a key is generated through a calculation that combines the hospital's first private key with the insurance company's second public key. The specific format and type of the key depend on the encryption algorithm and application scenario. Examples of key formats suitable for use in the present disclosure include a matrix, a string, Privacy-Enhanced Mail (PEM) format, binary data, a JSON Web Key, and certificates, but are not limited to these.

In step S230, the FHIR authorized data to be transmitted is encrypted using the key generated in step S220. This step involves implementing a specific algorithm and applying a key to encrypt the FHIR authorized data, thereby producing ciphered FHIR authorized data that unauthorized individuals cannot easily decipher. Only the party possessing the corresponding private key (i.e., the insurance institution with the second private key) can decrypt the data and restore it to its readable original content.

After the FHIR authorized data is encrypted (i.e., the ciphered FHIR authorized data), to ensure that it originates from a specific sender (e.g., a hospital), in step S240, a signature is added to the ciphered FHIR authorized data using the sender's first private key before transmission, thereby creating the signed FHIR authorized data. Since the first private key is not publicly available, the receiver (e.g., an insurance company) can verify the sources of the received data by checking whether it contains the signature generated by the first private key.

In step S250, the signed FHIR authorized data is verified by using the first public key to validate the signature, thereby restoring the ciphered FHIR authorized data. If the received data does not originate from the specific sender (e.g., a specific hospital), the signature cannot be verified and rescinded with the first public key, and the ciphered FHIR-authorized data cannot be restored back.

Proceed to step S260, the second private key held by the service-provider (e.g., insurance company) is further combined with the first public key held by the sender (e.g., a hospital) to generate a key as same as the key produced in step S220. The key is then used to decrypt the ciphered FHIR authorized data, thereby restoring it to a human-readable format and retrieving the FHIR authorized data.

Alternatively or optionally, the method 200 further comprises the step of transmitting the ciphered FHIR authorized data of step S230, the signed FHIR authorized data of step S240, or a combination thereof to the blockchain database 120 after the ciphered FHIR authorized data and/or the signed FHIR authorized data is formed.

According to one alternative or additional embodiment of the present disclosure, after step S230, the method 200 further comprises steps of calculating the ciphered FHIR authorized data to produce a hash value; and transmitting the hash value to the blockchain database 120.

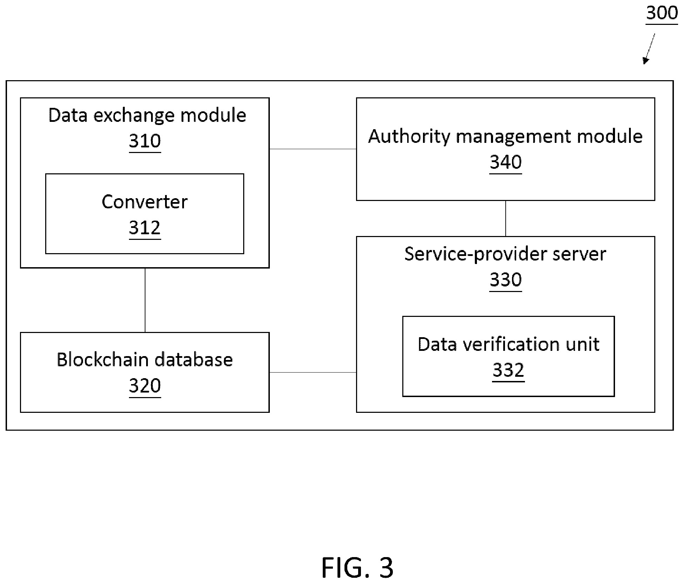

FIG. 3 is a schematic diagram of a system 300 constructed according to an alternative embodiment of the present disclosure. Similar to the system 100, the system 300 includes, in its structure, a data exchange module 310 including a converter 312, a blockchain database 320, a service-provider server 330, and an authority management module 340, all of which are communicatively connected and configured to perform the same functions as described for system 100. Thus, the details are omitted herein for the sake of brevity. The system 300 differs from the system 100 in that the service-provider server 330 of the system 300 (*i.e.,* the insurance company) includes an additional data verification unit 332, which is communicatively connected to the blockchain database 320 and is used to calculate a checksum from the ciphered FHIR authorized data. The checksum is subsequently compared with the hash value to verify their consistency. When the checksum matches the hash value, it indicates that the received ciphered FHIR authorized data is identical to the data encrypted in step S230 (*i.e.,* the FHIR authorized data). Conversely, if the checksum does not match the hash value, it suggests that the received ciphered FHIR authorized data has been tampered with during the data exchange, compromising its authenticity and making it ineligible as a basis for providing claims services.

By virtue of the above configuration, the present system for medical data exchange and instructions programmed therein for executing the encryption-decryption processes ensures the security and confidentiality during the exchange.

### 2.2 Method for exchanging medical data and settling medical claims

A second aspect of the present disclosure aims to provide a method for exchanging medical data between medical and insurance institutions, and settling medical claims for a client using the client's raw medical data stored in a server via the present system (e.g., the systems 100 or 300 aforementioned), thereby enabling the provision of medical claims services to the client.

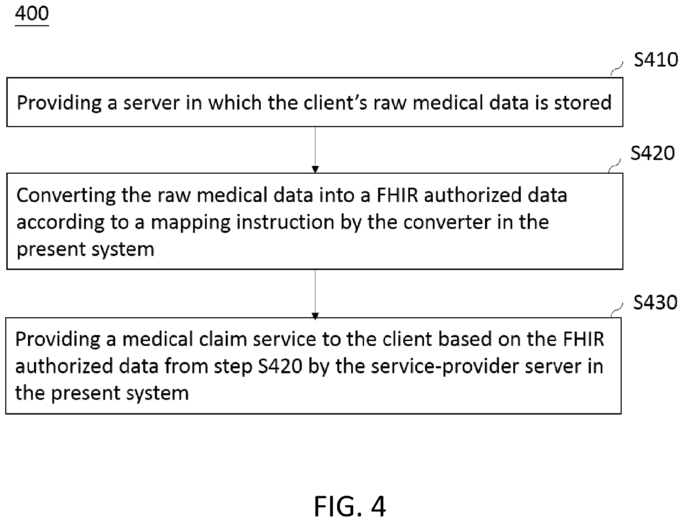

Accordingly, referring to FIG. 4, which is a flow chart illustrating a method 400 according to one embodiment of the present disclosure. The method 400 comprises the steps of,
S410: providing a server in which the client's raw medical data is stored;
S420: converting the raw medical data into a FHIR authorized data in response to a mapping instruction by use of the converter in the present system; and
S430: providing a medical claim service to the client based on the FHIR authorized data from step S420 by the service-provider server in the present system.

Specifically, the method 400 begins with storing the client's raw medical data for the claims service in the server of a medical institution (e.g., a hospital or healthcare organization). The structure and content of the raw medical data are recorded in a first format including but not limited to XML, JSON, RDF, and Turtle (step 410).

In step 420, the raw medical data stored in the server is transmitted to the present system (100, 300), which is communicatively connected to the server. As set forth in section 2.1, the present system includes a data exchange module (110, 310), a blockchain database (120, 320), a service-provider server (130, 330), and an authority management module (140, 340), all of which are interconnected. The data exchange module (110, 310) receives the raw medical data from the server and, using the built-in converter (112) to convert the raw medical data into FHIR authorized data in response to mapping instructions. This process facilitates the exchange of medical data and the provision of insurance claims services to the client.

According to embodiments of the present disclosure, the mapping instructions are a set of instructions or specifications created by defining the mapping between the items in the raw medical data and fields in the FHIR standard data. This mapping is based on the fields and structure of the FHIR standard data (i.e., the hierarchical relationships among resources and elements in the FHIR standard data), and is preferably defined by the user.

After generating the FHIR authorized data, it can be transmitted to the blockchain database (120, 320) for storage and to the service-provider server (130, 330), respectively, for subsequent claims processing (step S430).

According to embodiments of the present disclosure, to enhance the security and privacy during data exchange, the authority management module of the present system executes authority management for the FHIR authorized data transmitted between the data exchange module (110, 310), the blockchain database (120, 320), and the service-provider server (130, 330). Specifically, the authority management module is programmed with instructions to execute a procedure comprising steps of: (a) receiving a first public key and a first private key from the server, and a second public key and a second private key from the service-provider server, respectively; (b) combining the first private key and the second public key from step (a), thereby producing a key; (c) encrypting the FHIR authorized data with the key produced in step (b), thereby producing a ciphered FHIR authorized data; (d) providing a signature to the ciphered FHIR authorized data of step (c) by use of the first private key, thereby producing a signed FHIR authorized data; (e) verifying the signed FHIR authorized data of step (d) by use of the first public key to validate and rescind the signature, thereby restoring the ciphered FHIR authorized data of step (c); and (f) decrypting the ciphered FHIR authorized data of step (e) with the key of step (b) to obtain the FHIR authorized data that has been decrypted, wherein the key is produced by combining the second private key and the first public key of step (a).

By performing the encryption, decryption, and signature procedures as set forth above, the security during data transmission is ensured, as the original content cannot be deciphered even if intercepted by a third party. The procedures also verify that the received medical data is from the designated source, such as a designated medical institution.

According to some preferred embodiments of the present disclosure, after the step (c), the authority management module is further programmed with instructions to execute the following steps: generating a hash value composed of characters of a specific length by applying specific mathematical function to the ciphered FHIR authorized data, and transmitting the hash value to the blockchain database. In one preferred embodiment, the service-provider server is further equipped with a data verification unit, which is communicatively connected to the blockchain database. The data verification unit is used to compute a checksum of hash values from the ciphered FHIR authorized data received from the data exchange module or the blockchain database. The checksum is then compared with the hash value stored in the blockchain database. Consistent results indicate that he received ciphered FHIR authorized data obtained in step (f) is the same as the ciphered FHIR authorized data generated in step (c), otherwise the FHIR authorized data may have been altered or tempered with during the exchange process.

To sum up, by converting medical data into FHIR standard format and applying encryption-decryption and signature procedures for both the data sender and receiver, the system and method for medical data exchange and medical claims service according to the present disclosure achieve data interoperability, security, and privacy between medical institutions and insurance companies. The advantages of the present disclosure not only include efficiently shortening the time required for claims processing but also improving the client experience.

It will be understood that the above description of embodiments is given by way of example only and that various modifications may be made by those with ordinary skill in the art. The above specification, examples, and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the spirit or scope of this invention.

## Claims

1. A system for implementing medical data exchange and medical claim settlement via a Fast Healthcare Interoperability Resources (FHIR) standard data, communicatively connected to a server having a plurality of raw medical data of a first format stored thereon, the system comprises:
a data exchange module configured to receive the plurality of raw medical data from the server and convert them into a plurality of FHIR authorized data in response to a mapping instruction via a converter built therein;
a blockchain database communicatively connected to the data exchange module and configured to store the plurality of FHIR authorized data;
a service-provider server communicatively connected to the data exchange module and the blockchain database, and is configured to provide a claim settlement based on the plurality of FHIR authorized data; and
an authority management module communicatively connected to the data exchange module and the service-provider server, and is programmed with instructions to execute a method for managing each of the FHIR authorized data transmitted among the data exchange module, the blockchain database, and the service-provider server, wherein the method comprises,
(a) receiving a first public key and a first private key from the server, and a second public key and a second private key from the service-provider server, respectively;
(b) combining the first private key and the second public key of step (a) to produce a key;
(c) encrypting each of the FHIR authorized data with the key produced in step (b) to produce a ciphered FHIR authorized data;
(d) providing a signature to the ciphered FHIR authorized data of step (c) by use of the first private key, thereby producing a signed FHIR authorized data;
(e) verifying the signed FHIR authorized data of step (d) by use of the first public key to validate and rescind the signature, thereby restoring the ciphered FHIR authorized data of step (c); and
(f) decrypting the ciphered FHIR authorized data of step (e) with the key of step (b) to obtain the FHIR authorized data that has been decrypted, wherein the key is produced by combining the second private key and the first public key of step (a);
wherein the FHIR standard data has a second format that differs from the first format of the plurality of raw medical data.

2. The system of claim 1, wherein the mapping instruction is produced through self-defining the FHIR standard data of the second format.

3. The system of claim 2, wherein the first format is an extensible markup language (XML) format, and the second format is a JavaScript object notation (JSON) format.

4. The system of claim 3, wherein the converter is an XML to JSON converter.

5. The system of claim 1, wherein after step (c) and/or after step (d) of the method, the method further comprises transmitting the ciphered FHIR authorized data of step (c), the signed FHIR authorized data of step (d), or a combination thereof to the blockchain database.

6. The system of claim 5, wherein after step (c) of the method, the method further comprises:
calculating the ciphered FHIR authorized data to produce a hash value; and
transmitting the hash value to the blockchain database.

7. The system of claim 6, wherein the service-provider server further comprises a data verification unit communicatively connected to the blockchain database and is configured to calculate the ciphered FHIR authorized data to produce a checksum and to verify the consistency between the hash value and the checksum.

8. A computer-implemented method for exchanging medical data and settling medical claims for a client using the client's raw medical data stored in a server via the system of claim 1, the computer-implemented method comprises,
(i) receiving a raw medical data of the client from the server, wherein the raw medical data has a first format;
(ii) converting the raw medical data to a FHIR authorized data in response to a mapping instruction by use of the converter of the system of claim 1, wherein the FHIR standard data has a second format that differs from the first format of the raw medical data, and the FHIR authorized data is stored in the blockchain database of the system of claim 1; and
(iii) providing a medical claim service to the client based on the FHIR authorized data of step (ii) by the service-provider server of the system of claim 1;
wherein the FHIR authorized data is processed according to instructions programmed within the authority management module of the system of claim 1 as the FHIR authorized data is transmitted among the data exchange module, the blockchain database, and the service-provider server of the system of claim 1 by executing a procedure comprising steps of:
(a) receiving a first public key and a first private key from the server, and a second public key and a second private key from the service-provider server of the system of claim 1, respectively;
(b) combining the first private key and the second public key of step (a)to produce a key;
(c) encrypting the FHIR authorized data with the key produced in step (b) to produce a ciphered FHIR authorized data;
(d) providing a signature to the ciphered FHIR authorized data of step (c) by use of the first private key, thereby producing a signed FHIR authorized data;
(e) verifying the signed FHIR authorized data of step (d) by use of the first public key to validate and rescind the signature, thereby restoring the ciphered FHIR authorized data of step (c); and
(f) decrypting the ciphered FHIR authorized data of step (e) with the key of step (b) to obtain the FHIR authorized data that has been decrypted, wherein the key is produced by combining the second private key and the first public key of step (a).

9. The method of claim 8, wherein the mapping instruction of step (ii) is produced through self-defining the FHIR standard data with the second format.

10. The method of claim 8, wherein the authority management module is further programmed with instructions to execute the steps of transmitting the ciphered FHIR authorized data of step (c), the signed FHIR authorized data of step (d), or a combination thereof to the blockchain database after the ciphered FHIR authorized data and/or the signed FHIR authorized data are produced.

11. The method of claim 10, wherein after step (c), the authority management module is further programmed with instructions to execute the steps of
calculating the ciphered FHIR authorized data to produce a hash value; and
transmitting the hash value to the blockchain database.

12. The method of claim 11, wherein the service-provider server further comprises a data verification unit communicatively connected to the blockchain database and configured to calculate the ciphered FHIR authorized data to produce a checksum, and to verify the consistency between the hash value and the checksum.
